(19) Europäisches Patentamt
European Patent Office
Office européen des brevets

(11) Publication number: **0 121 284 B1**

(12) **EUROPEAN PATENT SPECIFICATION**

(45) Date of publication of patent specification: **11.03.92**

(51) Int. Cl.⁵: **C07D 249/08**, A01N 43/64

(21) Application number: **84200456.6**

(22) Date of filing: **14.12.81**

(60) Publication number of the earlier application in accordance with Art.76 EPC: **0 054 431**

Divisional application 90121117.7 filed on 14/12/81.

(54) **( + )-Triazolylpentenol derivative, its production and use as herbicide and/or plant growth regulant.**

(30) Priority: **15.12.80 JP 177704/80**
**22.12.80 JP 182407/80**

(43) Date of publication of application:
**10.10.84 Bulletin 84/41**

(45) Publication of the grant of the patent:
**11.03.92 Bulletin 92/11**

(84) Designated Contracting States:
**BE CH DE FR GB IT LI NL**

(56) References cited:
**EP-A- 0 015 639**
**DE-A- 2 920 437**
**GB-A- 2 046 260**

(73) Proprietor: **SUMITOMO CHEMICAL COMPANY, LIMITED**
**Kitahama 4-chome 5-33**
**Chuo-ku Osaka 541(JP)**

(72) Inventor: **Funaki, Yuji**
**10-3-356, Sonehigashinocho-2-chome**
**Toyonaka-shi(JP)**
Inventor: **Yoneyoshi, Yukio**
**30-16, Hiyoshidai-3-chome**
**Otsu-shi(JP)**
Inventor: **Ishiguri, Yukio**
**Sumitomokagaku-Mefuryo 14-7, Mefu-2-chome**
**Takarazuka-shi(JP)**
Inventor: **Izumi, Kazuo**
**Sumitomokagaku-Mefuryo 14-7, Mefu-2-chome**
**Takarazuka-shi(JP)**

(74) Representative: **Goldin, Douglas Michael et al**
**J.A. KEMP & CO. 14, South Square Gray's Inn**
**London WC1R 5EU(GB)**

EP 0 121 284 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to an optically active triazolyl alcohol derivative having an optical activity of (+) and the E-geometric configuration which may be represented by formula (I),

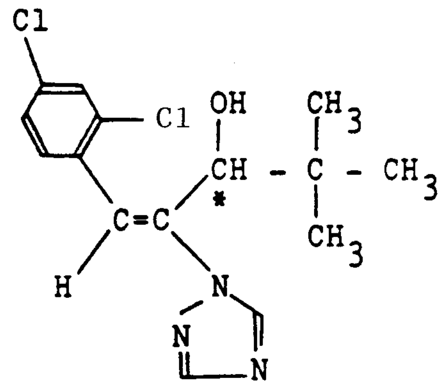

(I)

wherein the asterisk indicates an asymmetric carbon atom and salts thereof, a process for preparing same, and plant growth regulatory and herbicidal compositions containing the same as active ingredient and the use of this compound in the preparation of plant growth regulatory and herbicidal preparations.

The present application is divided from European Patent Application no 81305873.2 granted as EP O 054 431

The racemic triazolyl alcohol derivatives and their excellent fungicidal, plant growth regulatory, and herbicidal activities have already been described in Japanese Patent Application "Kokai" (Laid-open) No. 124,771/1980, Japanese Patent Application No. 100,547/1980, DE-A- 29 20 437 and GB-A-2 046 460.

The triazolyl alcohol derivative of formula (I) has optical isomers due to the asymmetric carbon atom (*C). The triazolyl alcohol derivative (I) having an optical activity of (+), as above referred to, is an optical isomer which shows an optical rotation of (+), as measured in chloroform with sodium D line, and is hereinafter referred to as the (+)-triazolyl alcohol derivative. On the other hand, another isomer which shows an optical rotation of (-) is hereinafter referred to as the (-)-triazolyl alcohol derivative. Salts of the triazolyl alcohol derivative are also included in the scope of this invention. These salts are salts with the plant-physiologically tolerable acids. Examples of such acids include hydrogen halides such as hydrobromic acid, hydrochloric acid and hydroiodic acid; carboxylic acids such as acetic acid, trichloroacetic acid, maleic acid and succinic acid; sulfonic acids such as p-toluenesulfonic acid and methanesulfonic acid; nitric acid, sulfuric acid, and phosphoric acid. These salts are obtained in a customary manner.

The present inventors examined in detail the usefulness of (-)- or (+)-triazolyl alcohol derivatives (I) obtained by the process of this invention. Upon comparison of (-)-, (+)- and racemic triazolyl alcohol derivatives with one another, it was found that the fungicidal activity falls in the order : (-)-triazolyl alcohol derivative > racemic triazolyl alcohol derivative > (+)-triazolyl alcohol derivative, whilst the plant growth regulatory activity and the herbicidal activity are in the order : (+)-triazolyl alcohol derivative > racemic triazolylalcohol derivative > (-)-triazolyl alcohol derivative. In short, the present inventors have discovered an entirely new fact that a (-)-triazolyl alcohol derivative exhibits an excellent fungicidal activity, while a (+)-triazolyl alcohol derivative exhibits an excellent plant growth regulatory activity and herbicidal activity.

The present invention contributes much to the cultivation of plants in the fields of agriculture and horticulture. For instance, application of a more active chemical is connected to adequate application of a smaller amount of the chemical, which leads to the improvement in economics of the processes of manufacture, transportation and field application and to the expectation of minimizing the environmental pollution as well as the improvement in safety.

As stated above, the (+)-triazolyl alcohol derivative can be utilized as a plant growth regulator to regulate the growth of useful plants. For instance, it can be applied to keep the rice plant, wheat and barley, lawn grass, hedge plants and fruit trees from spindle growth and also to effect dwarfing of potted garden plants such as chrysanthemum, pansy, poinsettia, azalea and rhododendron. In rice cropping and wheat or barley cropping, the lodging of rice, wheat or barley plants caused by excessive application of fertilizers or by gales often presents an important problem. By applying the (+)-triazolyl alcohol derivative to rice, wheat or barley at a proper stage of growth, spindling can be suppressed so that the plant height may be suitably controlled to keep effectively the plant from lodging. In the cultivation of chrysanthemum in pot, application

of the compound results in a reduction in the stem length without injurious effect on the flower, thus improving the commercial value of the plant.

Further, the (+)-triazolyl alcohol derivative exhibits a strong herbicidal activity against gramineous weeds such as barnyard millet, large crabgrass and green foxtail; cyperaceous weeds such as purple nutsedge; broad-leaved weeds in upland field such as green amaranth, fat hen, common purslane and common chickweed; annual and perennial weeds in paddy field such as barnyardgrass, monochoria, spike-flowered rotala, Dapatrium junceum, bulrush and slender spikerush.

When applied to an upland field, the (+)-triazolyl alcohol derivative exhibits a strong activity against principal weeds and is effective for the pre-emergence treatment of soil as well as for the foliage treatment in an early stage of growth. The compound has tremendous advantages in that it has no harmful effect on principal crops such as rice, soybean, cotton, corn, peanut, sunflower and sugar beet and can be safely used also for the vegetables such as lettuce, radish and tomato. The compound, therefore, is useful for weeding in a variety of situations such as in grain fields, vegetable gardens, orchards, lawns, pastures, tea fields, mulberry fields, rubber plantations, forest lands and non-cultivated fields. It was found, moreover, that the compound is highly nontoxic to mammals and fishes and is substantially harmless to agriculturally useful crops.

The methods for preparing the (+)-triazolyl alcohol derivative include those used in preparing conventional optically active substances such as asymmetric reduction and resolution of the diastereomer obtained from a racemate and an optically active reactive compound. These methods are described below in detail.

(1) Preparation by asymmetric reduction.

The racemate of the present compound is obtained by reducing a ketone compound represented by the general formula (II) with a metal-hydrogen complex such as lithium aluminum hydride ($LiAlH_4$) or sodium borohydride ($NaBH_4$) [Japanese Patent Publication "Kokai" (Laid-open) No. 124,771/1980].

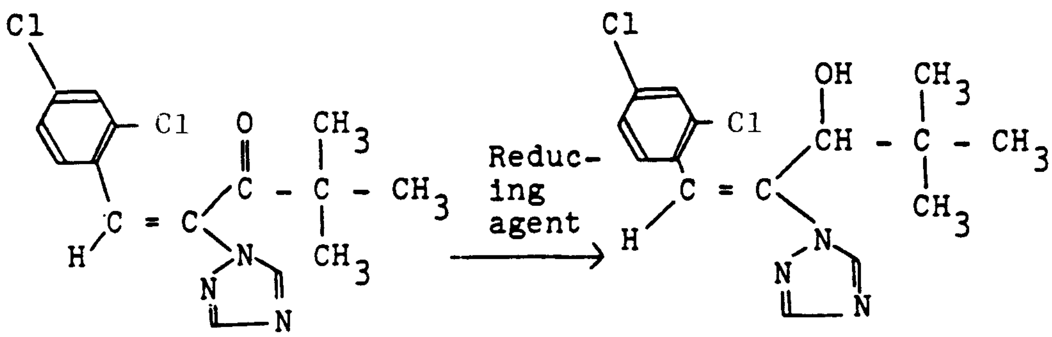


(II) (I) Racemate

Asymmetric reduction is commonly conducted by utilizing the enantioselective reaction which takes place when a ketone compound (II) is reduced with a chiral metal-hydrogen complex. A few of such procedures are described below.

(a) It is a general practice to use as the chiral metal-hydrogen complex a reducing agent of the chiral modified lithium aluminum hydride type formed by the partial decomposition of lithium aluminum hydride with an optically active alcohol [Literature: Tetrahedron Letters, Vol. 29, 913 (1973); Bull. Soc. Chim. Fr., 1968, 3795; J. Org. Chem., 38 (10), 1973; Tetrahedron Letters, Vol. 36, 3165 (1976)].

Among examples of the optically active alcohols used in this invention as asymmetric source, may be cited (-)-menthol, (-)-borneol, (-)-N-methylephedrine, and (-)-2-N,N-dimethylamino-1-phenylethanol. It is of course possible to use the (-)-optically active forms of other optically active alcohols including alkaloids, carbohydrates and amino alcohols such as, for example, quinine, cis-Myrtanol, 2-N-benzyl-N-methylamino-1-phenylethanol and 4-dimethylamino-3-methyl-1,2-diphenyl-2-butanol. The formation of a chiral modified lithium aluminum hydride reducing agent using an optically active alcohol as asymmetric source can be effected by adding 1 to 3 equivalent ratio of the optically active alcohol to one equivalent ratio of lithium aluminum hydride suspended in a suitable solvent. It is a general practice to use as the solvent an ether such as diethyl ether, tetrahydrofuran or dioxane, though an aromatic hydrocarbon such as benzene or toluene or an aliphatic hydrocarbon such as n-hexane or n-pentane may also be used.

(b) It is sometimes advantageous to use as the chiral metal-hydrogen complex a chiral modified lithium aluminum hydride reducing agent formed by the reaction of one equivalent ratio of an optically active alcohol, 2 equivalent ratio of a N-substituted aniline represented by the general formula (III),

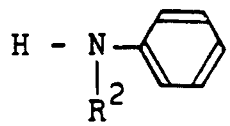

(III)

wherein $R^2$ is a lower alkyl group or a phenyl group, and one equivalent ratio of lithium aluminum hydride[literature: Tetrahedron Letters, Vol. 21, 2753 (1980)]. The optically active alcohol used as asymmetric source in this invention is exemplified by the (-)-optically active forms of amino alcohols such as N-substituted ephedrine, for example, (-)-N-methylephedrine and 2-N,N-disubstituted-amino-1-phenylethanol, for example (-)-2-N,N-dimethylamino-1-phenylethanol. As to the N-substituted aniline, a desirable result is obtained by use of a lower alkyl-substituted aniline such as N-methylaniline or N-ethylaniline, or diphenylamine. The preparation of such a chiral modified lithium aluminum hydride reducing agent can be achieved by suspending one equivalent ratio of lithium aluminum hydride ($LiAlH_4$) in a suitable solvent and admixing with one equivalent ratio of an optically active alcohol followed by 2 equivalent ratio of a N-substituted aniline. The solvent described above in (a) can be used likewise.

The asymmetric reduction is conducted by adding a ketone compound (II) dissolved in a suitable solvent to the chiral modified lithium aluminum hydride prepared as in (a) or (b) described above. The solvent is the same as described in (a). The reaction temperature is preferably 0 °C or below, though a temperature between -80 °C and the boiling point of the solvent can be used. After completion of the reaction, the complex compound is decomposed by the addition of a dilute aqueous acidic solution and the reaction mixture is purified by extraction, silica gel column chromatography or recrystallization to obtain the intended product.

(2) Preparation by resolution of diastereomers.

A method for resolving optical isomers by use of diastereomer esters formed from a racemic alcohol compound and an optically active reactive compound has been known (literature: Org. Reaction, Vol. 2, 380). A diastereomeric ester mixture (IV) is obtained by allowing a racemate of triazolyl alcohol compound (I) to react with a reactive derivative of optically active carboxylic acid in the presence of a base. The (+)-triazolyl alcohol derivative (I) is obtained by resolving said diastereomeric ester mixture by chromatography or fractional crystallization into the ester of the (+)-triazolyl alcohol and the ester of the (-)triazolyl alcohol, and decomposing the ester of the (+)- triazolyl alcohol.

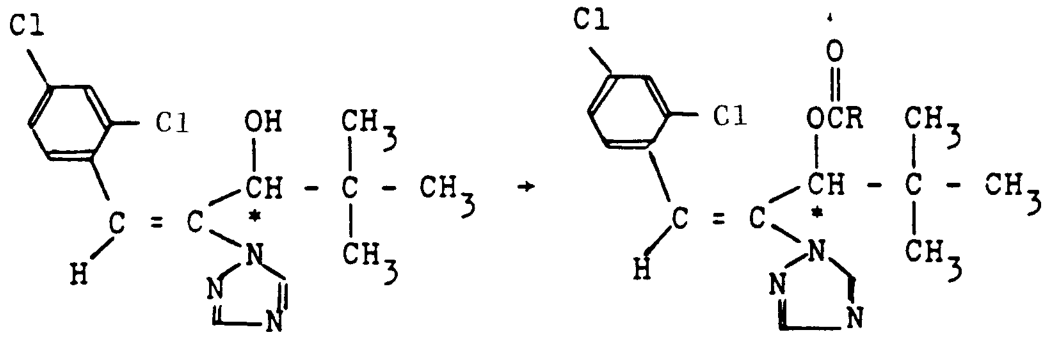

(I) Racemate (IV) Diastereomeric mixture

Resolution

→ (+)-Triazolyl alcohol ester → (+)-Triazolyl alcohol compound

→ (-)-Triazolyl alcohol ester → (-)-Triazolyl alcohol compound

(In the above formulae, the asterisk is as defined above.)

As examples of the optically active carboxylic acids for use in the esterification of racemate of the triazolyl alcohol (I), there are (-)-menthoxyacetic acid, (+)- or (-)-N-trifluoroacetylproline, (+)-camphoric acid, (+)- or (-)-mandelic acid, (+)- or (-)-2-phenylpropionic acid, (+)- or (-)-2-isopropyl-4'-chlorophenylacetic acid, (+)- or (-)-α-methoxy-α-trifluoromethylphenylacetic acid, (+)- or (-)-cis-chrysanthemic acid, and (+)- or (-)-transchrysanthemic acid. The reactive derivatives of these optically active carboxylic acids include acid halides and acid anhydrides. Generally, the optically active carboxylic acid is converted into an acid halide in a customary manner and allowed to react with the racemate of the triazolyl alcohol (I) to effect esterification. The reaction is conducted in a common inert solvent (e.g., acetone, acetonitrile, tetrahydrofuran, ethyl acetate, benzene, toluene, dichloromethane, chloroform and carbon tetrachloride) and in the presence of a dehydrohalogenation agent (e.g., triethylamine, N,N-dimethylaniline and pyridine). Generally, 1 to 5 moles of an acyl halide and a dehydrohalogenation agent are used for one mole of the triazolyl alcohol racemate (I). Pyridine behaves also as a solvent when used in excess. The reaction temperature is in the range of from room temperature to the boiling point of the solvent. It is of course possible to prepare the diastereomeric ester by using the anhydride of an optically active carboxylic acid.

When the diastereomeric mixture of a triazolyl alcohol ester (V) obtained as described above is crystallizable, it is resolved by repeated fractional crystallization, while if it is in oily form, the resolution is effected by column chromatography or high-speed liquid chromatography. The ester of the (+)-triazolyl alcohol ester thus formed is decomposed in the presence of a base such as sodium hydroxide or potassium hydroxide in a suitable solvent such as water or an aqueous organic solvent (ethanol or methanol is generally used) to obtain the (+)-triazolyl alcohol derivative (I).

In the field application of the compound of this invention obtained as described above, it may be used either alone without the addition of other ingredients or in mixtures with a carrier to make them more convenient for use as a herbicide and a plant growth regulator. Examples of the usual preparation forms include dust, wettable powder, oil spray, emulsion, tablet, granule, fine granule, aerosol and flowable. These preparations contain generally 0.1 to 95.0%, preferably 0.2 to 90.0% by weight of the active compound (including other active ingredients). A suitable application rate is 2 to 500 g/10 ares and a preferable concentration of the active ingredients for field application is 0.001 to 1.0%. However, the concentration may be suitably increased or decreased without sticking to the said range, because the application rate and the concentration depend on the type of preparation, application time of the year, method of application, site

of application and the type of crop to be treated.

For use as a herbicide and plant growth regulator, the present compound can be mixed with fungicides and insecticides, and even a synergetic effect is expectable from such a mixture. Examples of fungicides include N-(3,5-dichlorophenyl)-1,2-dimethylcyclopropane-1,2-dicarboximide, S-n-butyl S-p-tert-butylbenzyldithiocarbonimidate, 0,0-dimethyl 0-(2,6-dichloro-4-methylphenyl)-phosphorothioate, methyl 1-butylcarbamoyl-1H-benzimidazol-2-yl-carbamate, N-trichloromethylthio-4-cyclohexene-1,2-dicarboximide, cis-N-(1,1,2,2-tetrachloroethylthio)-4-cyclohexene-1,2-dicarboximide, Polyoxin, streptomycin, zinc ethylenebisdithiocarbamate, zinc dimethylthiocarbamate, manganese ethylenebisdithiocarbamate, bis(N,N-dimethylthiocarbamoyl)disulfide, tetrachloroisophthalonitrile, 8-hydroxyquinoline, dodecylguanidine acetate, 5,6-dihydro-2-methyl-1,4-oxathiin-3-carboxanilide, N'-dichlorofluoromethylthio-N,N-dimethyl-N'-phenylsulfamide, 1-(4-chlorophenoxy)-3,3-dimethyl-1-(1,2,4-triazol-1-yl)-2-butanone, 1,2-bis(3-methoxycarbonyl-2-thioureid)benzene, methyl N-(2,6-dimethylphenyl)-N-methoxyacetyl-2-methylglycinate, and aluminum ethylphosphite. Examples of the insecticides include organophosphorus insecticides such as 0,0-dimethyl 0-(4-nitro-3-methylphenyl)phosphorothioate, 0-(4-cyanophenyl) 0,0-dimethylphosphorothioate, 0-(4-cyanophenyl) 0-ethylphenylphosphonothioate, 0,0-dimethyl S-(1-ethoxycarbamoylmethyl)phosphorodithioate, 2-methoxy-4H-1,3,2-benzodioxaphosphorin-2-sulfide, 0,0-dimethyl S-(1-ethoxycarbonyl-1-phenylmethyl)-phosphorodithioate, and pyrethroid insecticides such as α-cyano-3-phenoxybenzyl 2-(4-chloro-phenyl)-isovalerate, 3-phenoxybenzyl 2,2-dimethyl-3-(2,2-dichlorovinyl)cyclopropanecarboxylate, and α-cyano-3-phenoxybenzyl 2',2'-dimethyl-3'-(2,2-dibromovinyl)cyclo-propanecarboxylate.

The invention is further illustrated below in detail with reference to Examples, Reference Examples, Test Examples. The Formulation Examples show typical formulations of triazolyl alcohols such as the compound of the invention. Examples of further reagents which may be used in producing the present compound can be seen in EP-A-0 054 431.

Reference Example 1

Synthesis of (E)-1-(2,4-dichlorophenyl)-2-(1,2,4-triazol-1-yl)-4,4-dimethyl-1-penten-3-ol racemate:

To 50 cc of a methanol solution containing 3.2 g of (E)-1-(2,4-dichlorophenyl)-2-(1,2,4-triazol-1-yl)-4,4-dimethyl-1-penten-3-one (melting point 92 - 93° C) characterized by the NMR spectrum shown below, was added 0.5 g of sodium borohydride, while cooling in ice. The mixture was then stirred at room temperature for 3 hours and 1 ml of acetic acid was added to effect decomposition. The organic layer was extracted with 100 ml of ethyl acetate and the extract was washed with 50 ml of a 5% aqueous hydrogen carbonate solution, and dried over anhydrous sodium sulfate. After removal of the solvent by distillation under reduced pressure, the residue was recrystallised from n-hexane to obtain 2.6 g of the captioned compound melting at 148 - 149° C.

The NMR spectra were shown below in terms of δ value measured on a deutero-chloroform solution.

(E)-1-(2,4-dichlorophenyl)-2-(1,2,4-triazol-1-yl)-4,4-dimethyl-1-penten-3-one:

8.30 (1H, singlet, triazole proton), 8.04 (1H, singlet, triazole proton), 7.45 (1H, multiplet, phenyl proton), 7.26 (2H, multiplet, phenyl proton), 7.22 (1H, singlet, olefin proton), 0.97 (9H, singlet, butyl proton).

(E)-1-(2,4-dichlorophenyl)-2-(1,2,4-triazol-1-yl)-4,4-dimethyl-1-penten-3-ol:

8.45 (1H, singlet, triazole proton), 7.97 (1H, singlet, triazole proton), 7.80 (3H, multiplet, phenyl proton), 6.80 (1H, singlet, olefin proton), 4.35 (2H, broad singlet, hydroxyl proton and hydroxyl group-bearing methyne proton), 0.63 (9H, singlet, tert-butyl proton).

Example 1

Synthesis of (+)-(E)-1-(2,4-dichlorophenyl)-2-(1,2,4-triazol-1-yl)-4,4-dimethyl-1-penten-3-ol by resolution of diastereomeric ester:

A mixture of 4 g of (±)-(E)-1-(2,4-dicholorophenyl)-2-(1,2,4-triazol-1-yl)-4,4-dimethyl-1-penten-3-ol and 8 g of (-)-menthoxyacetyl chloride was stirred in 50 cc of pyridine at 70° C for 7 hours. The reaction mixture was poured into 200 cc of ice water and extracted with 400 cc of ethyl acetate. The organic layer was washed successively with 0.5N hydrochloric acid, 200 cc of a saturated aqueous sodium hydrogencarbonate solution and 200 cc of ice-cooled water, then dried over anhydrous sodium sulfate, and concentrated in vacuo. The crude oily substance was purified by silica gel chromatography (150 g of silica gel; developing solvent: n-hexane/acetone = 30 : 1) to obtain 5 g of (±)-[(E)-1-(2,4-dichlorophenyl)-2-(1,2,4-triazol-1-yl)-4,4-dimethyl-1-penten-3-yl]-(-)-menthoxy acetate. Upon repetition of chromatography on another

silica gel column (250 g of silica gel; developing solvent: n-hexane/benzene/acetone = 20/20/1), the diastereomeric ester mixture gave 1.6 g of (-)-[(E)-1-(2,4-dichlorophenyl)-2-(1,2,4-triazol-1-yl)-4,4-dimethyl-1-penten-3-yl]-(-)-menthoxyacetate ($n_D^{28}$ 1.5172) as first eluate, 2 g of the diastereomeric ester mixture as second eluate, and 0.7 g of (+)-[(E)-1-(2,4-dichlorophenyl)-2-(1,2,4-triazol-1-yl)-4,4-dimethyl-1-penten-3-yl]-(-)-menthoxyacetate ($n_D^{28}$ 1.5102) as final eluate.

A mixture 0.7 g of (+)-[(E)-1-(2,4-dichlorophenyl)-2-(1,2,4-triazol-1-yl)-4,4-dimethyl-1-penten-3-yl]-(-)-menthoxyacetate with 20 cc of a 90% aqueous ethanol solution containing 0.1 g of potassium hydroxide was stirred at 25°C for one hour. The reaction mixture was poured into 200 cc of ice water and extracted with 300 cc of ethyl acetate. The organic layer was dried over anhydrous sodium sulfate and concentrated in vacuo. The resulting crude crystals were recrystallized from a carbon tetrachloride-n-hexane mixture to obtain 0.3 g of (+)-(E)-1-(2,4-dichlorophenyl)-2-(1,2,4-triazol-1-yl)-4,4-dimethyl-1-penten-3-ol: $[\alpha]_D^{24}$ + 26.0 (c = 1.0, $CHCl_3$); melting point 160 - 161°C.

Example 2

Synthesis of (+)-(E)-1-(2,4-dichlorophenyl)-2-(1,2,4-triazol-1-yl)-4,4-dimethyl-1-penten-3-ol by asymmetric reduction:

A chiral metal-hydrogen complex was formed dropwise adding over a period of 30 minutes 50 cc of an ethyl ether solution containing 3.05 g of (-)-N-methylephedrine, and the mixture was then stirred at room temperature for 15 minutes. To the mixture was added dropwise over a period of 30 minutes 20 cc of an ethyl ether solution containing 4.12 g of N-ethylaniline, and the resulting mixture was stirred at room temperature for 3 hours. To the resulting solution, was added at -70°C 30 cc of an ethyl ether solution containing 1.62 g of (E)-1-(2,4-dichlorophenyl)-2-(1,2,4-triazol-1-yl)-4,4-dimethyl-1-penten-3-one. The resulting mixture was stirred at -73°C for 5 hours under adiabatic conditions and left standing overnight at room temperature. To the mixture was added 60 cc of 2N hydrochloric acid to effect decomposition. The organic layer was washed successively with 100 cc of a saturated sodium hydrogencarbonate solution and 100 cc of ice-cooled water, dried over anhydrous sodium sulfate, and concentrated under reduced pressure to obtain 1.82 g of crude crystals. The crude crystals were recrystallized three times from a cyclohexane-methanol mixture to obtain 0.41 g of (+)-(E)-1-(2,4-dichlorophenyl)-2-(1,2,4-triazol-1-yl)-4,4-dimethyl-1-penten-3-ol: $[\alpha]_D^{24}$ + 29.2 (c = 1.0, $CHCl_3$); melting point 160 - 161°C. The NMR spectrum was identical with that of the racemate described in Reference Example 1.

The useful properties of the (+)-triazolyl alcohol derivative of this invention are illustrated below in detail with reference to some examples of tests performed on (+)-(E)-(2,4-dichlorophenyl)-2-(1,2,4-triazol-1-yl)-4,4-dimethyl-1-penten-3-ol (compound No. 1) obtained in Example 1 and (-)-(E)-1-(2,4-dichlorophenyl)-2-(1,2,4-triazol-1-yl)-4,4-dimethyl-1-penten-3-ol (compound No. 2) obtained as a by product in Example 1 and the racemate obtained in Reference Example 1 (compound No. 3).

Test Example 1

Inhibition of fungus growth:

A medium containing 5 g of polypeptone, 20 g of malt extract, 20 g of sucrose and 20 g of agar per liter of water heated to form a liquid. To the liquefied medium, was added a predetermined quantity of a diluted sample of the test compound in the form of emulsifiable concentrate so as to keep the concentration of each sample in the medium at a predetermined level. After thorough stirring, the medium was poured into a Petri dish to form an agar plate. After the agar had set, it was inoculated with a colony or a conidium suspention of the test fungus. The fungus species and the incubation period (the period of time from inoculation to observation) were as shown below. The incubation temperature was 20°C for Venturia inaequalis and 28°C for other fungi.

| Fungus species | Abbreviation | Incubation period (day) |
|---|---|---|
| Helminthosporium gramineum | Hg | 6 |
| Penicillium italicum | Pi | 6 |
| Venturia inaequalis | Vi | 7 |
| Valsa mali | Vm | 4 |
| Mycosphaerella melonis | Mm | 4 |
| Diaporthe citri | Dc | 6 |
| Ustilago nuda | Un | 6 |
| Verticillium albo-atrum | Va | 7 |
| Septoria tritici | St | 7 |
| Cercospora beticola | Cb | 7 |

The fungus growth inhibitory activity of the test compound was evaluated by the concentration which inhibits 90% of the mycelium growth ($ED_{90}$). As is seen from the results shown in Table 1, it was found that the (+) triazolyl alcohol derivative of this invention (compounds No. 1) shows markedly reduced antifungal spectra compared with (-)-triazolyl alcohol derivative and racemate (compounds No. 2 and No. 3).

Further test data showing the low antifungal activity of the present compound can be seen in EP-A-0 054 431.

Table 1. Concentration, in ppm, which inhibits 90% colony growth ($ED_{90}$).

| Fungus species | compound No. 2 | compound No. 1 | compound No. 3 |
|---|---|---|---|
| Hg | 1.5 | >25.0 | 6.0 |
| Pi | 0.2 | >25.0 | 1.0 |
| Vi | <0.1 | 25.0 | 0.1 |
| Vm | <0.1 | >25.0 | 0.1 |
| Mn | 0.2 | >25.0 | 1.0 |
| Dc | 1.0 | >25.0 | 5.0 |
| Un | 5.0 | >25.0 | 5.0 |
| Va | 0.4 | >25.0 | 1.3 |
| St | 0.1 | >25.0 | 0.4 |
| Cb | 0.4 | >25.0 | 1.5 |

The results of examination on the plant growth regulatory activity and the herbicidal activity are given below. It was shown that the activity of the (+)-triazolyl alcohol derivative is far higher than those of the racemate and (-)-triazolyl alcohol derivatives.

Test Example 2

Test on wheat for growth retardation:

A 85-ml plastic pot filled with sandy loam was seeded with 10 to 15 seeds of wheat (var. Chikugo No. 2), which had been soaked in an aqueous diluted liquor of the test compound in emulsifiable concentrate form, and cultivated at a controlled temperature of 18° to 23°C for 7 days. The leaf length was then

measured and the percentage elongation was obtained by comparison with the leaf length in the control plot.

$$\text{Elongation (\%)} = \frac{\text{Plant length in treated plot}}{\text{Plant length in control plot}} \times 100$$

The test results were as shown in Table 2. It was found that the plant growth retarding activities of the (-)-triazolyl alcohol derivative and the racemate were far lower than that of the (+)-triazolyl alcohol derivative.

Table 2

| Compound No. | Concentration of treating liquor (ppm) | Plant length (mm) | Elongation (%) |
|---|---|---|---|
| 2 | 12.5<br>3.1 | 133<br>138 | 96<br>100 |
| 1 | 12.5<br>3.1 | 75<br>95 | 54<br>69 |
| 3 | 12.5<br>3.1 | 90<br>105 | 65<br>76 |
| Control (untreated) | - | 138 | 100 |

Test Example 3

Dwarfing test on pot-mum:

Pot-mum (var. Paragon) was cultivated in a 4.8-inch clay pot filled with 500 g of an artificial soil composed of sea sand, mountain soil and peat. Two weeks after setting, the plant was pinched so as to undergo the triple stem training. Two weeks after pinching, when sprouting had already begun, the test compound diluted to a predetermined concentration was applied to the plant and the growth retarding effect was inspected 42 days after the treatment. The results were as shown in Table 3. The effect was evaluated in the following way: the difference between the plant height at the time of application of the chemical and the plant height on the 42nd day after the application is recorded and expressed as elongation index which is the percentage of said difference based on the similar difference in the untreated plot. The indices shown in Table 3 were mean values of three replications.

All of the test compounds showed inhibition of internode elongation and reduction in plant height, but the phytotoxicity such as necrosis or chlorisis was not observed and even the green color of leaves became deeper. The (+)-triazolyl alcohol derivative (compound No. 1) showed a far stronger dwarfing effect compared with the (-)-triazolyl alcohol derivative (compound No. 1) and a stronger effect compared with the racemate (compound No. 3).

Table 3. Dwarfing test on pot-mum.

| Compound No. | Concentration of treating solution (ppm) | Elongation index (%) |
|---|---|---|
| 1 | 200<br>100<br>50 | 48<br>79<br>91 |
| 2 | 200<br>100<br>50 | 90<br>101<br>103 |
| 3 | 200<br>100<br>50 | 63<br>95<br>101 |

Test Example 4

Test on apple seedling for growth retardation of current shoot:

An apple seedling (var. Golden Delicious) planted in a 18-cm clay pot was pruned and cultivated in a greenhouse. Three weeks after the emrgence of current shoots, the above-ground part of the plant was entirely treated with a liquid preparation of the test compound in predetermined concentration by means of a hand-sprayer. Fourteen days after the treatment, the lengths of current shoots were measured and the amount of elongation was obtained from the difference between said length and the length at the time of chemical treatment. Two pots of the plant were used for each chemical treatment and the growth length determined on 4 to 6 shoots. The results in average value were as shown in Table 4. The (+)-triazolyl alcohol derivative showed a far higher growth inhibitory activity as compared with the (-)-triazolyl alcohol derivative and racemate.

Table 4. Test on apple tree for growth retardation of current shoot.

| Compound No. | Concentration of treating liquor (ppm) | Elongation | |
|---|---|---|---|
| | | (mm) | (%) |
| 1 | 100<br>50<br>25 | 120<br>147<br>163 | 67<br>82<br>91 |
| 2 | 100<br>50<br>25 | 171<br>176<br>180 | 96<br>98<br>101 |
| 3 | 100<br>50<br>25 | 160<br>172<br>172 | 89<br>96<br>96 |
| Control (untreated) | - | 179 | 100 |

Test Example 5

Test on lawn grass for growth retardation:

A 1/5,000 - are Wagner pot filled with upland soil was seeded with seeds of lawn grass (var. Seaside grass). After covering with soil, the seeds were cultivated in a greenhouse. After one month, the grass was cut at a height of 1 cm from the ground level and both foliage and soil were treated with a predetermiend amount of the chemical preparation by using a hand sprayer. Two weeks after the treatment, the elongation of grass was examined, then the grass was cut again and the cultivation was continued for additional 4 weeks. The results of examination performed after two weeks (first examination) and four weeks (second examination) from the treatment were as shown in Table 5. As compared with the (-)-triazolyl alcohol derivative and racemate, the (+)-triazolyl alcohol derivative showed a far greater effect.

Test Example 6

Pot test of naked barley:

A 1/2,000 - are Wagner pot was filled with paddy soil of the plough layer, which passed through a wire screen having a square aperture of 1.5 x 1.5 cm. After applying as basal fertilizer a urea-base compound fertilizer at an application rate of $N/P_2O_5/K_2O$ = 1.3/1.3/1.3 g/pot, the pot was seeded with 12 seeds of naked barley (var. Hinodehadaka) on December 5. The seeds were cultivated in a greenhouse. When the seedling had emerged and grown to a height of several centimeters, the seedlings were thinned to 6 stumps per pot. At the beginning of internode elongation (Feb. 15), a predetermiend amount of the chemical preparation was sprayed over the soil surface and the cultivation was further continued until the harvest time (May 21) had reached when the plant height, number of ear, and the weight of husked barley were

measured. The results of examination were as shown in Table 6. Although all of the test compounds showed a dwarfing, tiller-promoting, and yield increasing effect, the (+)-triazolyl alcohol derivative exhibited much superior effect compared with the (-)-triazolyl alcohol derivative and a stronger dwarfing effect compared with the racemate.

Table 5. Test on lawn grass for growth retardation.

| Compound No. | Application rate (g/are) | Growth of lawn grass (cm) | | |
|---|---|---|---|---|
| | | 1st time | 2nd time | Total |
| 1 | 10<br>5<br>2.5 | 2.0<br>3.0<br>3.8 | 3.0<br>3.5<br>4.8 | 5.0<br>6.5<br>8.6 |
| 2 | 10<br>5<br>2.5 | 4.5<br>4.5<br>5.0 | 8.5<br>9.0<br>9.0 | 13.0<br>13.5<br>14.0 |
| 3 | 10<br>5<br>2.5 | 2.5<br>3.8<br>4.0 | 3.3<br>3.3<br>6.0 | 5.8<br>7.1<br>10.0 |
| Untreated | - | 5.0 | 9.0 | 14.0 |

Table 6. Pot test of naked barley.

| Compound No. | Application rate, active ingredient (g/are) | Number of ear (per pot) | Plant height (cm) | Weight of husked barley (g/pot) | Weight of husked barley (%) |
|---|---|---|---|---|---|
| 1 | 1 | 48.3 | 79.7 | 64.6 | 107 |
| | 5 | 53.3 | 72.3 | 62.4 | 103 |
| 2 | 1 | 45.0 | 86.1 | 59.6 | 98 |
| | 5 | 46.7 | 82.4 | 59.8 | 99 |
| 3 | 1 | 47.4 | 83.5 | 63.1 | 104 |
| | 5 | 49.0 | 80.1 | 62.4 | 103 |
| Untreated | - | 45.3 | 85.9 | 60.6 | 100 |

Test Example 7

Weed control test in upland soil:

A 1/1,000 - are Wagner pot was filled with a soil mixed with seeds of large crabgrass, green amaranth, and fat hen. A diluted aqueous emulsion containing a prescribed quantity of the test compound was applied by means of a hand sprayer to treat the soil surface. After the treatment, sugar beet seedlings (var.

Monohill) in fifth leaf age bred in a paper pot were transplanted to the Wagner pot and bred in a greenhouse. The weed control activity and phytotoxicity of the test compound were observed on 20th day from the treatment. The results of observation were as shown in Table 7.

The evaluation of weed control activity was performed by classifying the observed results into the following 6 grades of from 0 to 5.

The phytotoxicity was evaluated likewise.

| | Degree of weed control (%) |
|---|---|
| 0 | 0 - 9 |
| 1 | 10 - 29 |
| 2 | 30 - 49 |
| 3 | 50 - 69 |
| 4 | 70 - 89 |
| 5 | 90 - 100 |

Table 7. Weed control test in upland soil.

| Compound No. | Application rate (g/are) | Weed control activity | | | Phytotoxicity to sugar beet |
|---|---|---|---|---|---|
| | | Large crabgrass | Green amaranth | Fat hen | |
| 1 | 20 | 5 | 5 | 5 | 0 |
| | 10 | 4 | 5 | 5 | 0 |
| 2 | 20 | 3 | 3 | 3 | 0 |
| | 10 | 3 | 3 | 3 | 0 |
| 3 | 20 | 4 | 5 | 5 | 0 |
| | 10 | 4 | 4 | 5 | 0 |

Preparation Example 1 Dust.

Two parts of the compound, 88 parts of clay and 10 parts of talc are thoroughly mixed by grinding to form a dust preparation containing 2% of the active ingredient.

Preparation Example 2 Dust.

Three parts of the compound, 67 parts of clay and 30 parts of talc are thoroughly mixed by grinding to

form a dust preparation containing 3% of the active ingredient.

Preparation Example 3 Wettable powder.

Thirty parts of the compound, 45 parts of diatomaceous earth, 20 parts of white carbon, 3 parts of a wetting agent (sodium lauryl sulfate) and 2 parts of a dispersant (calcium ligninsulfonate) are thoroughly mixed by grinding to form a wettable powder preparation containing 30% of the active ingredient.

Preparation Example 4 Wettable powder.

Fifty parts of the compound, 45 parts of diatomaceous earth, 2.5 parts of a wetting agent (calcium alkylbenzenesulfonate) and 2.5 parts of a dispersant (calcium ligninsulfonate) are thoroughly mixed by grinding to form a wettable powder containing 50% of the active ingredient.

Preparation Example 5 Emulsifiable concentrate.

Ten parts of the compound, 80 parts of cyclohexanone and 10 parts of an emulsfier (polyoxyethylene alkylaryl ether) are mixed to form an emulsifiable concentrate containing 10% of the active ingredient.

Preparation Example 6 Granule.

Five parts by weight of the compound, 40 parts by weight of bentonite, 50 parts by weight of clay and 5 parts by weight of sodium ligninsulfonate are thoroughly mixed by grinding, then thoroughly milled together with water, granulated, and dried to form a granule preparation.

Preparation Example 7 Liquid.

0.05 Parts by weight of the compound, 1 part by weight of "Hymal 1009" (a surfactant produced by Matsumoto Yushi Co.), 1 part by weight of "Newcol 560" (a nonionic emulsifier), 2.5 parts by weight of cyclohexanone and 95.45 parts by weight of water are mixed to form a liquid preparation.

**Claims**

**1.** The triazolyl alcohol derivative of formula (I)

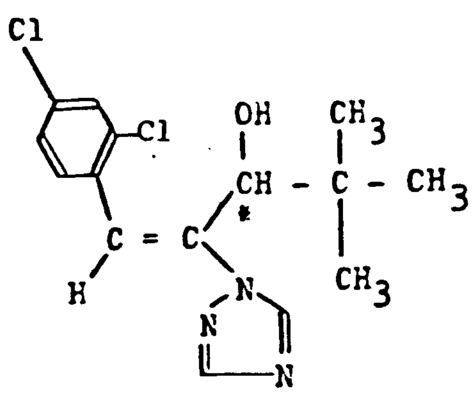

(I)

wherein the asterisk indicates an asymmetric carbon atom, having optical activity of (+) and the E-geometric configuration, and salts thereof.

**2.** A process for producing the triazolyl alcohol derivative defined in claim 1 which comprises asymmetrically reducing a triazolyl ketone derivative of formula (II)

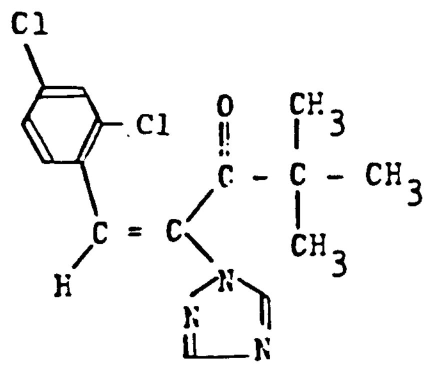

(II)

with a chiral reducing agent of the modified lithium aluminium hydride type formed by partial decomposition of lithium aluminium hydride with an optically active alcohol.

3. A process according to claim 2 wherein use is made of the chiral reducing agent of the modified lithium aluminium hydride type prepared from 1 equivalent of lithium aluminium hydride, 1 equivalent of the (-) optical isomer of an N-substituted ephedrine and 1 equivalents of an N-alkylaniline.

4. A process according to claim 2, wherein use is made of the chiral reducing agent of the modified lithium aluminium hydride type prepared from 1 equivalent of lithium aluminium hydride, 1 equivalent of the (-) optical isomer of 2-N,N-disubstituted amino-1-phenylethanol and 2 equivalents of an N-alkylaniline.

5. A process according to claim 2 wherein use is made of the chiral reducing agent of the modified lithium aluminium hydride type prepared from 1 equivalent of lithium aluminium hydride, 2 to 3 equivalents of (-)-menthol.

6. A process for producing the (+)-triazolyl alcohol derivative as defined in claim 1 which comprises reacting a racemate of a triazolyl alcohol derivative of formula (III)

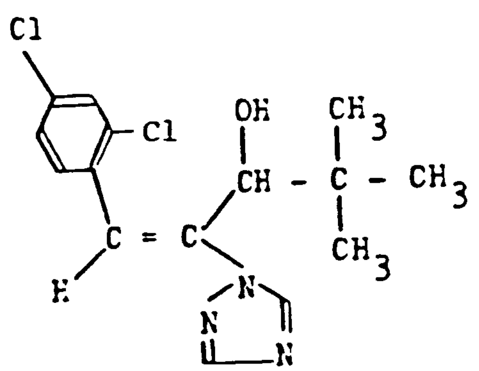

(III)

with a reactive derivative of an optically active carboxylic acid to form two diastereomeric esters, isolating the diastereomeric ester of the (+)-triazolyl alcohol and hydrolysing the isolated ester.

7. A process according to claim 6 wherein (-)- or (+)-menthoxyacetyl chloride is used as the reactive derivative of an optically active carboxylic acid.

8. A plant growth regulatory and herbicidal composition containing a plant physiologically acceptable inert carrier and as an active ingredient the triazolyl alcohol derivative of formula (I)

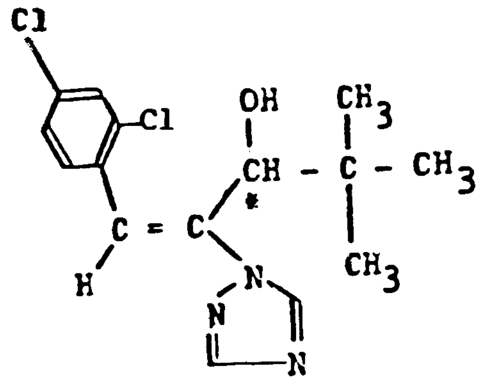

(I)

wherein the asterisk indicates an asymmetric carbon atom, having an optical activity of (+) and the E-geometric configuration.

**9.** A method for controlling plant growth, which comprises applying a plant growth regulatory composition according to claim 8 to the plant.

**10.** A method for killing weeds, which comprises applying a herbicidal composition according to claim 8 to the weed.

**11.** The use of the triazolyl alcohol derivative of formula (I)

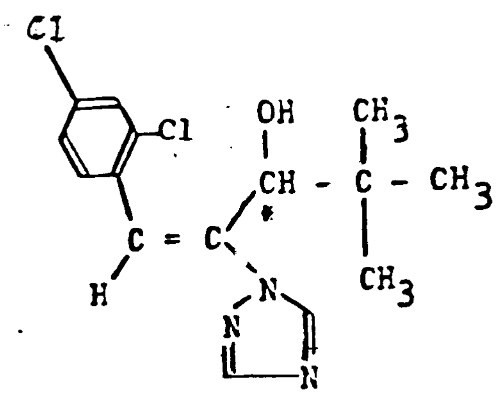

(I)

wherein X is hydrogen or chlorine and the asterisk indicates an asymmetric carbon atom, having an optical activity of (+) and the E-geometric configuration in the manufacture of a plant regulatory preparation for treatment of plants.

**12.** The use of the triazolyl alcohol derivative of formula (I)

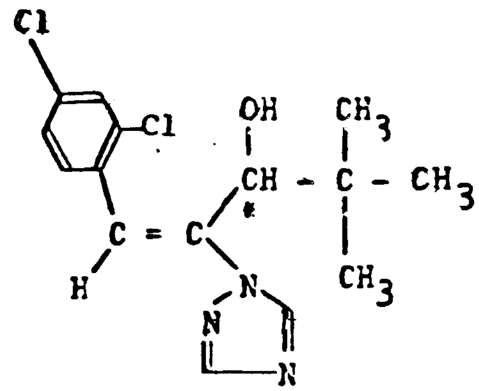

(I)

wherein the asterisk indicates an asymmetric carbon atom, having an optical activity of (+) and the E-geometric configuration in the manufacture of a herbicidal preparation for treatment of weeds.

**Revendications**

1. Dérivé d'alcool triazolylique de formule (I)

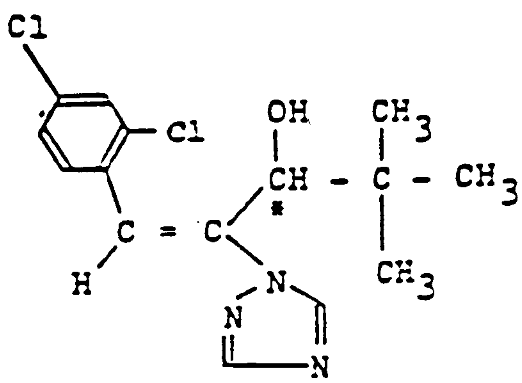

(I)

dans laquelle l'astérisque indique un atome de carbone asymétrique, ayant une activité optique (+) et la configuration géométrique E, et ses sels.

2. Procédé de production du dérivé d'alcool triazolylique défini dans la revendication 1, qui consiste à réduire de façon asymétrique le dérivé de cétone triazolylique de formule générale (II)

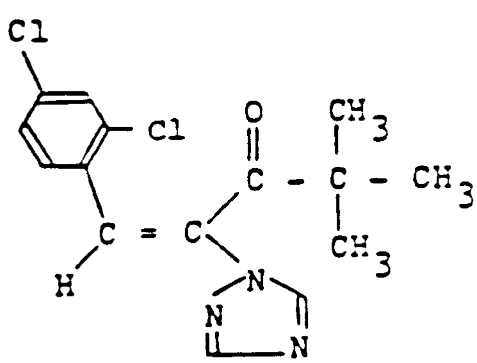

(II)

avec un agent réducteur asymétrique du type hydrure de lithium et d'aluminium modifié formé par décomposition partielle de l'hydrure de lithium et d'aluminium par un alcool optiquement actif.

3. Procédé selon la revendication 2, dans lequel on utilise comme agent réducteur asymétrique du type hydrure de lithium et d'aluminium modifié celui préparé à partir de 1 équivalent d'hydrure de lithium et d'aluminium, de 1 équivalent de l'isomère optique (-) d'une éphédrine N-substituée et de 2 équivalents d'une N-alkylaniline.

4. Procédé selon la revendication 2, dans lequel on utilise comme agent réducteur asymétrique du type hydrure de lithium et d'aluminium modifié celui préparé à partir de 1 équivalent d'hydrure de lithium et d'aluminium, de 1 équivalent de l'isomère optique (-) d'un 2-amino-1-phényléthanol N,N-disubstitué et de 2 équivalents d'une N-alkylaniline.

5. Procédé selon la revendication 2, dans lequel on utilise comme agent réducteur asymétrique du type hydrure de lithium et d'aluminium modifié celui préparé à partir de 1 équivalent d'hydrure de lithium et d'aluminium et de 2 à 3 équivalents de (-)-menthol.

6. Procédé de production du dérivé d'alcool triazolylique (+) défini dans la revendication 1, qui consiste à faire réagir un racémate d'un dérivé d'alcool triazolylique de formule (III)

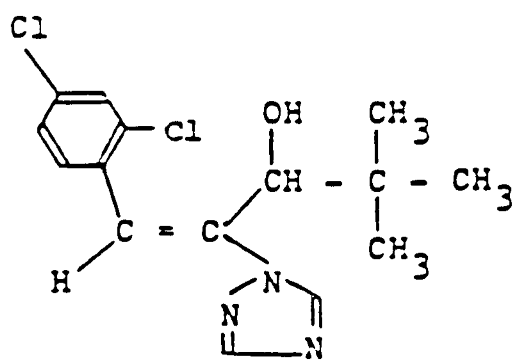

(III)

avec un dérivé réactif d'un acide carboxylique optiquement actif pour former deux esters diastéréoisomères, à isoler l'ester diastéréoisomère de l'alcool triazolylique (+) et à hydrolyser l'ester isolé.

7. Procédé selon la revendication 6, dans lequel on utilise le chlorure de (+)-menthoxyacétyle comme dérivé réactif d'un acide carboxylique optiquement actif.

8. Composition de régulation de la croissance de plantes et herbicide, contenant un support inerte acceptable sur le plan phytophysiologique et, comme ingrédient actif, le dérivé d'alcool triazolylique de formule (I)

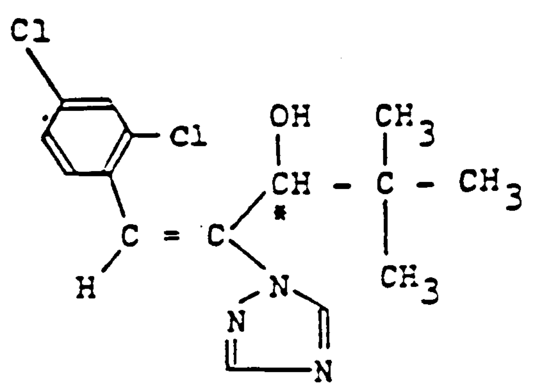

(I)

dans laquelle l'astérisque indique un atome de carbone asymétrique, ayant une activité optique (+) et la configuration géométrique E.

**9.** Méthode de contrôle de la croissance de plantes, qui consiste à appliquer aux plantes une composition de régulation de la croissance des plantes selon la revendication 8.

**10.** Méthode de destruction de mauvaises herbes, qui consiste à appliquer aux mauvaises herbes une composition herbicide selon la revendication 8.

**11.** Utilisation du dérivé d'alcool triazolylique de formule (I)

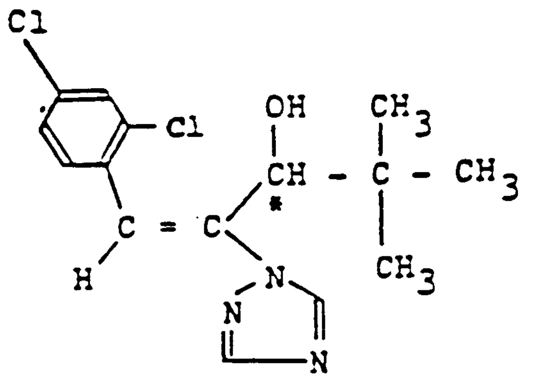

(I)

dans laquelle l'astérisque indique un atome de carbone asymétrique, ayant une activité optique (+) et la configuration géométrique E, dans la fabrication d'une préparation de régulation de la croissance des plantes pour le traitement de plantes.

**12.** Utilisation du dérivé d'alcool triazolylique de formule (I)

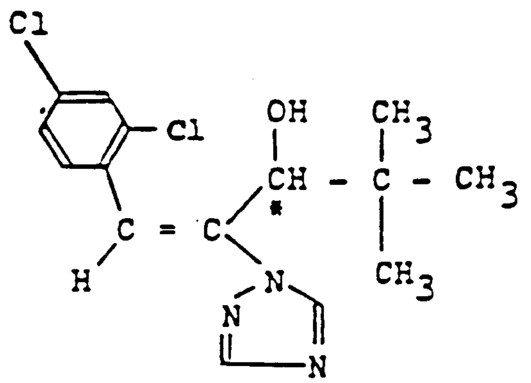

(I)

dans laquelle l'astérisque indique un atome de carbone asymétrique, ayant une activité optique (+) et la configuration géométrique E, dans la fabrication d'une préparation herbicide pour le traitement des mauvaises herbes.

**Patentansprüche**

**1.** Triazolylalkoholderivat der Formel (I)

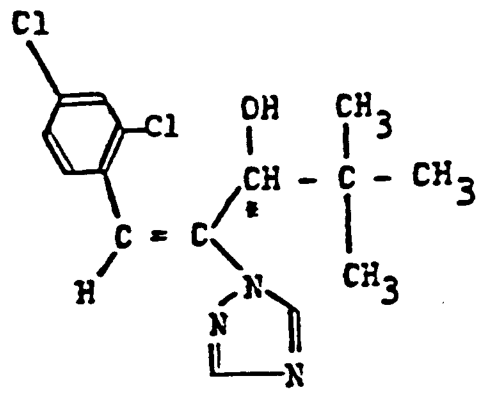

(I)

in der der Stern ein asymmetrisches Kohlenstoffatom kennzeichnet, das die optische Aktivität (+) und die geometrische Konfiguration E besitzt, und Salze davon.

**2.** Verfahren zur Herstellung des Triazolylalkoholderivats nach Anspruch 1, umfassend die asymmetrische Reduktion eines Triazolylketonderivats der Formel (II)

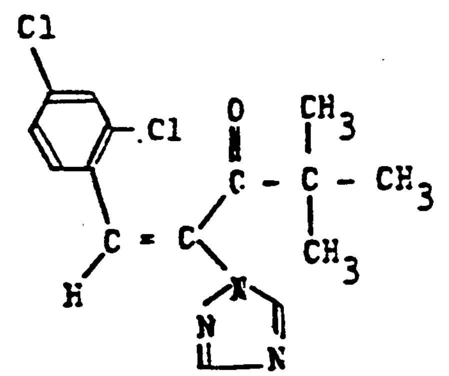

(II)

mit einem chiral reduzierenden Mittel des modifizierten Lithiumaluminiumhydrid-Typs, hergestellt durch teilweise Zersetzung von Lithiumaluminiumhydrid mit einem optisch aktiven Alkohol.

**3.** Verfahren nach Anspruch 2, wobei von einem chiral reduzierenden Mittel des modifizierten Lithiumaluminiumhydrid-Typs Gebrauch gemacht wird, das aus 1 Äquivalent Lithiumaluminiumhydrid, 1 Äquivalent des optischen (-) Isomers von N-substituiertem Ephedrin und 1 Äquivalent eines N-Alkylanilins hergestellt wurde.

**4.** Verfahren nach Anspruch 2, wobei von einem chiral reduzierenden Mittel des modifizierten Lithiumaluminiumhydrid-Typs Gebrauch gemacht wird, das aus 1 Äquivalent Lithiumaluminiumhydrid, 1 Äquivalent des optischen (-) Isomers von 2-N,N-disubstituierten Amino-1-phenylethanol und 2 Äquivalenten eines N-Alkylanilins hergestellt wurde.

**5.** Verfahren nach Anspruch 2, wobei von dem chiral reduzierenden Mittel des modifizierten Lithiumaluminiumhydrid-Typs Gebrauch gemacht wird, das aus 1 Äquivalent Lithiumaluminiumhydrid und 2 bis 3 Äquivalenten (-)-Menthol hergestellt wurde.

**6.** Verfahren zur Herstellung des (+)-Triazolylalkoholderivats nach Anspruch 1, umfassend die Umsetzung eines Razemats eines Triazolylalkoholderivats der Formel (III)

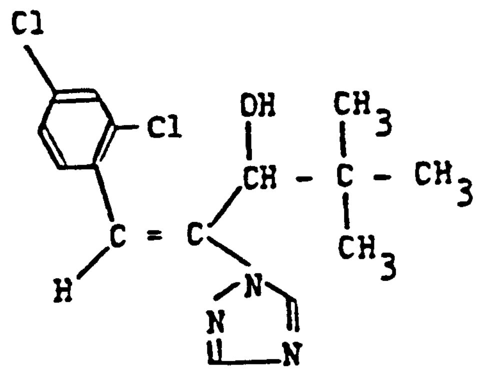

(III)

mit einem reaktiven Derivat einer optisch aktiven Carbonsäure zu zwei diastereomeren Estern, Gewinnung der diastereomeren Esters des (+)-Triazolylalkohols und Hydrolysieren des gewonnenen Esters.

**7.** Verfahren nach Anspruch 6, wobei (+)-Menthoxyacetylchlorid als reaktives Derivat einer optisch aktiven Carbonsäure verwendet wird.

**8.** Pflanzenwachstumsregulator und herbizides Mittel, enthaltend einen pflanzenphysiologisch verträglichen inerten Träger und als Wirkstoff das Triazolylalkoholderivat der Formel (I)

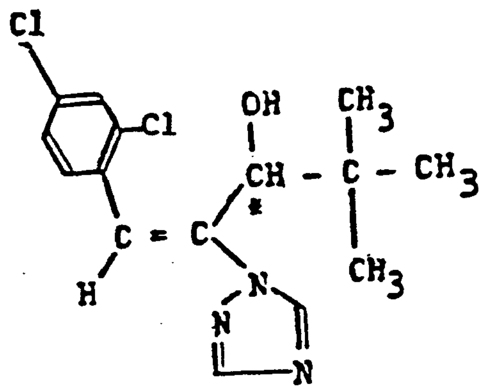

(I)

in der der Stern ein asymmetrisches Kohlenstoffatom kennzeichnet, das die optische Aktivität (+) und die geometrische Konfiguration E besitzt.

**9.** Verfahren zur Wachstumsbeeinflussung, umfassend die Anwendung eines pflanzenwachstumsregulierenden Mittels nach Anspruch 8 auf die Pflanze.

**10.** Verfahren zur Bekämpfung von Unkraut, umfassend die Anwendung eines herbiziden Mittels nach Anspruch 8 auf das Unkraut.

**11.** Verwendung des Triazolylalkoholderivats der Formel (I)

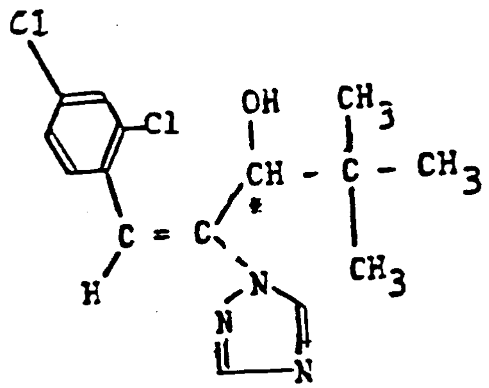

(I)

in der X ein Wasserstoffatom oder ein Chloratom bedeutet und der Stern ein asymmetrisches Kohlenstoffatom kennzeichnet, das die optische Aktivität (+) und die geometrische Konfiguration E aufweist, bei der Herstellung eines pflanzenregulierenden Mittels zur Behandlung von Pflanzen.

**12.** Verwendung des Triazolylalkoholderivats der Formel (I)

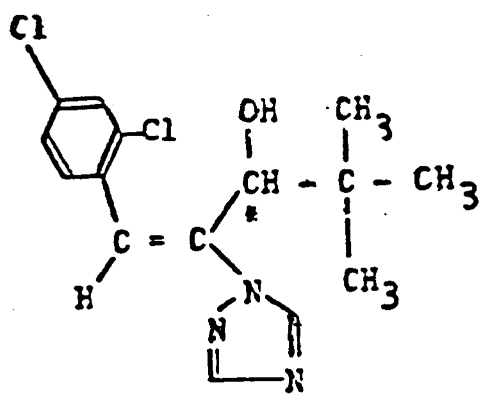

(I)

in der der Stern ein asymmetrisches Kohlenstoffatom kennzeichnet, das die optische Aktivität (+) und die geometrische Konfiguration E aufweist, bei der Herstellung eines herbiziden Mittels zur Unkrautbekämpfung.